# EUROPEAN PATENT SPECIFICATION

(11) **EP 0 646 389 B1**
(45) Date of publication and mention of the grant of the patent: **02.02.2000**
(21) Application number: 94113162.5
(22) Date of filing: 23.08.1994
(51) Int. Cl.: A61N 1/05

(54) **Device for explantation of an electrode**
Anordnung zur Explantation einer Elektrodenvorrichtung
Dispositif pour l'explantation d'une électrode

(30) Priority: 24.09.1993 SE 9303122
(43) Date of publication of application: 05.04.1995
(73) Proprietor: Pacesetter AB, 171 95 Solna (SE)
(72) Inventor: Lindegren, Ulf, S-122 35 Enskede (SE); Neubauer, Heinz, S-175 70 Järfälla (SE); Guerola, Modesto, ES-08007 Barcelona (ES)
(74) Representative: Lettström, Richard Wilhelm

(56) References cited:
- WO-A-91/19532
- WO-A-93/00130
- DD-A- 233 304
- US-A- 3 754 555
- US-A- 4 471 777
- US-A- 4 574 800
- US-A- 4 943 289
- "Chronic Transvenous Pacemaker Lead Removal Using a Unique, Sequential Transvenous System", The American Journal of Cardiology, Vol. 66, 16.10.90, p. 964-966

## Description

Die Erfindung bezieht sich auf eine Anordnung zur Explantation einer implantierten Elektrodenvorrichtung, bestehend aus einer intravaskulär oder intrakardial applizierten Elektrodenvorrichtung mit einem Elektrodenkabel mit bestimmter Länge und Ausendurchmesser und mit einem Elektrodenkopf, der am distalen Ende des Elektrodenkabels angebracht ist.

Eine in einem Patienten implantierte Elektrodenvorrichtung, z.B. eine Herzschrittmacherelektrode, wird nach einiger Zeit von Bindegewebe umschlossen, das das Elektrodenkabel derart verankert, dass es schwer und sogar gefährlich sein kann, nur an dem proximalen Ende des freigelegten Elektrodenkabels zu ziehen, da gesundes Gewebe im Bereich der Verankerungsstelle im Herzen losgerissen oder auf andere Weise zerstört werden kann. Mit Verankerungsstelle sind in der Regel der Elektrodenkopf am Elektrodenkabel, aber auch Fixiervorrichtungen in Form von z.B. Flossen oder Widerhaken gemeint, die unmittelbar hinter dem Elektrodenkopf angebracht sind und die bereits nach einer verhältnismässig kurzen Zeit von Bindegewebe umschlossen werden. Auch das Elektrodenkabel kann zum Teil durch fibröses Gewebe mit der umgebenden Venenwand verbunden sein. Daher wählt der Arzt häufig die Möglichkeit, das Elektrodenkabel implantiert zu lassen und dieses durch eine weitere Elektrodenvorrichtung zu ersetzen. In bestimmten Fällen, wenn z.B. mehrere ausgediente, abgeschnittene Elektrodenkabel über eine Vene im Herzen appliziert sind oder, wenn eine Entzündung entsteht oder das Risiko, dass die Elektrodenkabel eine Vene oder die Herzwand penetrieren, vorhanden ist, muss oder müssen wenigstens ein oder mehrere Elektrodenkabel entweder durch einen operativen Eingriff oder mit Hilfe einer Explantationsanordnung entfernt werden.

Aus der PCT-Anmeldung DE91/00490 mit der Publikationsnummer W091/19532 und in der US-PS 4 574 800 sind Explantationsvorrichtungen der eingangs genannten Art bekannt. Sämtliche hier beschriebenen Vorrichtungen bestehen aus mandrinförmigen Organen, deren distale Enden entweder mit nach hinten gerichteten Widerhaken oder mit radial expandierbaren Elementen versehen sind. Sämtliche Organe sind auch in einen Kanal der Elektrodenvorrichtung, der für einen Mandrin vorgesehen ist, einschiebbar. Wenn das mandrinförmige Organ eingeschoben ist, so dass dessen distales Ende sich unmittelbar hinter dem Elektrodenkopf befindet, richten sich die Widerhaken bzw. die Elemente in radialer Richtung derart auf, dass sie gegen die Innenwand des Kanals zu liegen kommen. Bei einer Explantation des Elektrodenkabels zieht der Arzt am Organ, was wie eingangs beschrieben mitsichführen kann, dass gesundes Gewebe im Bereich der Stellen, die mit dem Elektrodenkabel verbunden sind, beschädigt werden kann.

Eine Schneidvorrichtung ist aus DD-A-0 233 304 bekannt, die aus einem hülsenförmigen Zylinder besteht, in dem ein Kolben angeordnet ist, der mittels eines Schub- und Zugelementes, das axial aus dem Zylinder herausgeführt ist, in dem Zylinder in axialer Richtung hin- und herbewegbar und zum Teil aus dem Zylinder herausschiebbar und wieder hineinziehbar ist. Ein hakenförmiges Teil ist vorgesehen an der von den Schub- und Zugelement abgewandten Seite des Kolbens. Dieses Teil weist im Innern der Wölbung eine schräge Schneide auf, die mit einer Gegenkante des Zylinders in Wirkbeziehung gelangt.

Diese Schneidvorrichtung wird durch eine Einführungshülse bis an die Abtrenstelle des Elektrodenkabels herangeführt. Das Elektrodenkabel wird mit dem Haken an der Abtrennstelle eingefangen und durchtrennt.

Eine weitere Explantationsvorrichtung der eingangs genannten Art ist durch den Artikel in der Fachzeitschrift "The American Journal of Cardiology", Band 66 vom 15. Oktober 1990, Seiten 964-966 mit dem Titel "Chronic Transvenous Pacemaker Lead Removal Using a Unique, Sequential Transvenous System" bekannt. Diese Vorrichtung weist zwei flexible Rohre auf, bei denen das eine Rohr auf das andere aufgeschoben ist, so dass diese Rohre gemeinsam einen teleskopförmigen Katheter bilden. Der Katheter ist auf das Elektrodenkabel aufschiebbar und kann dadurch das Elektrodenkabel von der Venenwand losschneiden. Der Innendurchmesser des Katheters ist lediglich etwas grösser als der Aussendurchmesser des Elektrodenkabels, was bedeutet, dass eine sehr hohe Reibung zwischen dem Kabel und dem Katheter gegeben ist, was wiederum bedeutet, dass es keinesfalls problemlos ist, diesen langen Katheter gegenüber dem eventuell etwas längeren Kabel zu verschieben. Ausserdem muss der teleskopförmige Katheter, der über eine längere Strecke seine Eigenschaft aufschiebbar zu sein beibehalten soll, eine verhältnismässig hohe Wandstärke aufweisen, was wiederum bedeutet, dass die Flexibilität nicht besonders hoch sein kann. Der beschriebene Katheter löst auch nicht die Spitze des Elektrodenkopfes von der Herzwand.

Der Erfindung liegt die Aufgabe zugrunde, eine Anordnung der eingangs genannten Art zur Explantation einer Elektrodenvorrichtung zu schaffen, die auf eine sehr einfache und sichere Weise die Oberfläche des Elektrodenkabels von fibrösem Gewebe freilegen kann.

Diese Aufgabe ist erfindungsgemäss dadurch gelöst, dass die Anordnung aus einer im Vergleich zur Länge des Elektrodenkabels relativ kurzen und steifen ersten Hülse besteht, deren Innendurchmesser etwas grösser als der Aussendurchmesser des Elektrodenkabels ist, wobei die eine Stirnseite der Hülse eine Schneide bildet und wobei die Hülse mit von der Schneide abgewandten ersten Steuermitteln in Form eines Mandrins fest verbunden ist, mit dessen Hilfe die Hülse auf das Elektrodenkabel aufgeschoben und an der implantierten Elektrodenvorrichtung entlang verschoben werden kann. Nachdem das proximale Ende der implantierten Elektrodenvorrichtung vom Herzschrittmacher getrennt worden ist, wird dieses Ende abgeschnitten, so dass die Hülse auf das Elektrodenkabel aufgeschoben werden kann. Dadurch, dass hier das Kabel im Unterschied zur Verwendung einer Explantationsvorrichtung in Form eines Katheters für den Arzt erreichbar ist, kann er an dem proximalen Ende leicht ziehen, so dass das Kabel zusammen mit der Vene gerade gezogen wird. Dadurch, dass die Hülse verhältnismässig kurz und das Kabel verhältnismässig gerade ist, kann die Hülse mit Hilfe des Mandrins sehr leicht an dem Elektrodenkabel entlang verschoben werden, wobei die Schneide der Hülse eventuelles fibröses Gewebe, das das Kabel z.B. an der Innenwand der Vene oder an der Herzwand fixiert, abschneidet. Die Schneide der Hülse kann auch die herausragenden Fixiervorrichtungen, wenn diese aus einem Kunststoffmaterial hergestellt sind, abschneiden.

In einer vorteilhaften Weiterbildung der Erfindung wird eine zweite Hülse vorgeschlagen, die auf die erste Hülse aufschiebbar ist,wobei die eine Stirnseite der zweiten Hülse mit federnden in der Weise vorgebogenen Schneidemitteln versehen ist, dass diese in einer ersten Lage gegen die Aussenwand der ersten Hülse anliegen und in einer zweiten Lage gegeneinander gedrückt werden, wobei die zweite Hülse mit von den Schneidemitteln abgewandten zweiten Steuermitteln verbunden ist, mit deren Hilfe die zweite Hülse entlang der ersten Hülse verschoben werden kann, so dass die Schneidemittel von der einen zu der anderen Lage verschoben werden können. Hierdurch wird erreicht, dass auch die Spitze des Elektrodenkopfes, bei einer Verschiebung der zweiten Hülse von einer ersten in einer zweiten Lage, von fibrösem Gewebe losgeschnitten werden kann. Vorher ist die erste und die zweite Hülse selbstverständlich bis zum distalen Ende des Elektrodenkabels vorgeschoben worden.

Vorzugsweise besteht auch mindestens eine Hülse aus Metall. Hierdurch wird erreicht, dass die Schneide an der ersten Hülse und die Schneidemittel der zweiten Hülse sehr scharf gemacht werden können.

Weitere Vorteile und Einzelheiten ergeben sich aus den Unteransprüchen.

Die Erfindung ist nachfolgend anhand eines in den Zeichnungen dargestellten Ausführungsbeispiels näher erläutert. Es zeigen:
- FIG 1: eine Seitenansicht einer ersten Hülse nach der Erfindung,
- FIG 2: eine Seitenansicht einer zweiten Hülse nach einer vorteilhaften Weiterbildung der Erfindung,
- FIG 3: eine Seitenansicht der ersten und der zweiten Hülse nach der FIG 1 und 2, bei der die zweite Hülse auf die erste Hülse aufgeschoben ist, wobei die Hülsen ausserdem auf einem Elektrodenkabel angebracht sind und
- FIG 4 und 5: Hülsen nach der FIG 3 in verschiedenen gegenseitigen Positionen und in verschiedenen Lagen auf dem Elektrodenkabel

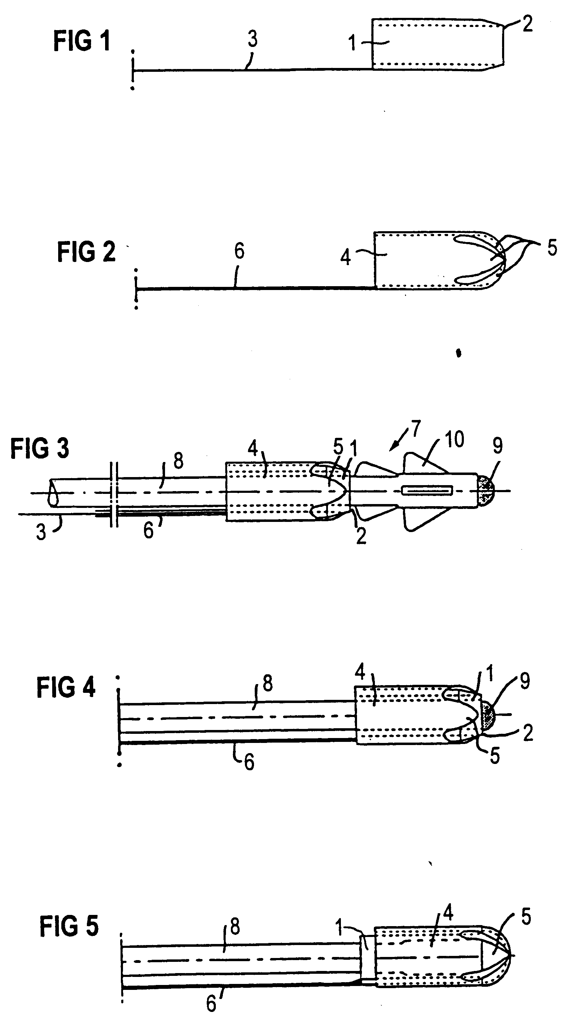

In der FIG 1 ist eine Anordnung zur Explantation einer hier nicht gezeigten, insbesondere einer intravaskulär oder intrakardial plazierten Elektrodenvorrichtung dargestellt. Die Anordnung besteht aus einer im Vergleich zur Länge des Kabels der Elektrodenvorrichtung relativ kurzen und steifen ersten Hülse 1, deren Innendurchmesser etwas grösser als der Aussendurchmesser des Elektrodenkabels 8 ist. Die eine Stirnseite der Hülse 1 ist abgefast und bildet eine Schneide 2. Die Hülse 1 ist ausserdem mit einem von der Schneide 2 abgewandten Mandrin 3, der vorzugsweise aus rostfreiem Stahl besteht, verbunden.

In der FIG 2 ist eine zweite Hülse 4 gezeigt, deren Länge etwa der Länge der ersten Hülse 1 entspricht und deren eine Stirnseite mit später beschriebenen federnden, vorgebogenen Schneidemitteln 5 versehen ist, die über den Umfang der Hülse 4 gleich verteilt sind. Die Hülse 4 ist ausserdem mit von den Schneidemitteln 5 abgewandten Steuermitteln 6 in Form eines verhältnismässig dünnen Rohres verbunden.

In der FIG 3 ist gezeigt, dass der Innendurchmesser der zweiten Hülse 4 derart dimensioniert ist, dass diese auf die erste Hülse 1 aufschiebbar und auf dieser verschiebbar ist. In dieser Lage der zweiten Hülse 4 drücken die Enden der Schneidemittel 5 gegen das abgefaste Teil der Hülse 1. Auf diese Weise ragen die vorgebogenen Schneidemittel 5 nicht von der Hülse 4 heraus, was vorteilhaft ist, da der Aussendurchmesser der Explantationsvorrichtung so klein wie möglich sein soll. Diese FIG soll auch darstellen, dass, wenn die zweite Hülse 4 auf die erste Hülse 1 aufgeschoben ist, der Mandrin 3 im Rohr 6 steckt. In der FIG ist ferner gezeigt, dass der Mandrin 3 für die erste Hülse 1 länger ist als das Rohr 6 für die zweite Hülse 4.

Vor einer Explantation einer intrakardial plazierten Elektrodenvorrichtung 7, bestehend aus dem Elektrodenkabel 8 und einem Elektrodenkopf 9, der am distalen Ende des Elektrodenkabels 8 angebracht ist, dessen proximales Ende freigelegt ist, wird in der Regel das Verbindungs- und Abdichtungsteil am Elektrodenkabel 8 abgeschnitten, da der Aussendurchmesser dieses Teils grösser als der Aussendurchmesser des übrigen Elektrodenkabels 8 ist. Nun kann der Arzt an dem proximalen Ende des Elektrodenkabels 8 ziehen, so dass dieses und auch die Vene, in der das Elektrodenkabel 8 geführt ist, gerade gestreckt werden, wobei die Hülsen 1 und 4 mit Hilfe des Mandrins 3 entlang dem Elektrodenkabel 8 verschoben werden können, wobei die Schneide 2 der Hülse 1 eventuelles Bindegewebe von der Oberfläche des Elektrodenkabels 8 abschneidet. In der FIG 3 sind die Hülsen 1 und 4 in einer Lage gezeigt, in der diese bis zu einer Anzahl flossenförmiger Fixiermittel 10 aus Kunststoff, die unmittelbar hinter dem Elektrodenkopf 8 angebracht sind, vorgeschoben sind. Derartige Fixiermittel 10 sind meistens von fibrösem Gewebe umgeben und sitzen häufig im Trabekelwerk der Herzwand sehr fest. Daher kann der Arzt, indem er die Hülsen 1 und 4 entlang des Elektrodenkabels verschiebt, mit Hilfe der Schneide 2 auch die Fixiermittel 10 von der Oberfläche des Elektrodenkabels 8 abschneiden.

In der FIG. 4 sind die Hülsen 1 und 4 in einer Lage gezeigt, in der sie unmittelbar hinter dem Elektrodenkopf 9 gebracht sind, was bedeutet, dass das gesamte Elektrodenkabel 8 von fibrösem Gewebe frei ist. Wenn der Arzt in dieser Lage der Hülsen 1 und 4 das Rohr 6 gegenüber dem Mandrin 3 vorschiebt, wird auch die zweite Hülse 4 gegenüber der ersten Hülse 1 verschoben, wobei die Schneidemittel 5 der Hülse 4 gegeneinander gedrückt werden und in der Weise die Spitze des Elektrodenkopfes 9 von fibrösem Gewebe losschneiden. Eine solche Lage ist in der FIG 5 dargestellt. Nun ist also die gesamte Elektrodenvorrichtung 7 von Bindegewebe frei und kann sie aus dem Herzen und der Vene des Patienten herausgezogen werden. Da die Hülsen 1 und 4 vorzugsweise aus Metall bestehen, können die Schneide 2 und die Schneidemittel 5 sehr scharf gemacht werden.

Der Mandrin 3 der ersten Hülse 1 braucht nicht unbedingt in dem Rohr 6 für die zweite Hülse 4 angebracht sein, sondern diese können voneinander getrennt sein.

Eine sehr einfache Explantationsvorrichtung ist auch gegeben, indem lediglich die erste Hülse 1 in beschriebener Weise verwendet wird, um Gewebe vom Elektrodenkabel 8 abzuschneiden, z.B., wenn der Arzt zu wissen glaubt, dass die Spitze des Elektrodenkopfes 9 aus irgend einem Grund nicht richtig mit Bindegewebe an der Herzwand fest verbunden ist.

### Bezugzeichenliste

- 1: erste Hülse
- 2: Schneide
- 3: Mandrin, Steuermittel
- 4: zweite Hülse
- 5: Schneidemittel
- 6: Steuermittel, Rohr
- 7: Elektrodenvorrichtung
- 8: Elektrodenkabel
- 9: Elektrodenkopf
- 10: Fixiermittel

## Claims

1. Arrangement for explanting an implanted electrode device (7) comprising an electrode device (7) which is applied intravascularly or inside the heart and has an electrode cable (8) of a certain length and outside diameter and an electrode head (9), which is mounted on the distal end of the electrode cable, wherein the arrangement comprises a stiff first sleeve (1) which is relatively short in comparison with the length of the electrode cable (8), the inside diameter of which first sleeve is slightly greater than the outside diameter of the electrode cable (8), wherein one end face of the sleeve (1) forms a cutting edge (2) and wherein the sleeve (1) is securely connected to first control means (3) in the form of a mandrin that face away from the cutting edge (2), with the aid of which control means the sleeve (1) can be slid on to the electrode cable (8) and moved along the implanted electrode device (7).

2. Arrangement according to claim 1, characterised in that the control means (3) consist of stainless steel.

3. Arrangement according to claim 1 or 2, characterised by a second sleeve (4) which can be slid on to the first sleeve (1), wherein one end face of the second sleeve (4) is provided with spring-mounted cutting means (5) which are pre-bent in such a way that in a first position the said cutting means rest against the outside wall of the first sleeve (1) and in a second position are pressed against each other, wherein the second sleeve (4) is connected to second control means (6) that face away from the cutting means (5), with the aid of which control means the second sleeve (4) can be moved along the first sleeve (1), so that the cutting means (5) can be moved from one position into the other position.

4. Arrangement according to claim 3, characterised in that the cutting means (5) are distributed evenly over the circumference of the second sleeve (4).

5. Arrangement according to claim 3 or 4, characterised in that the second control means (6) are formed from a tube.

6. Arrangement according to one of claims 3 to 5, characterised in that at least one sleeve (1, 4) consists of metal.

## Patentansprüche

1. Anordnung zur Explantation einer implantierten Elektrodenvorrichtung (7), bestehend aus einer intravaskulär oder intrakardial applizierten Elektrodenvorrichtung (7) mit einem Elektrodenkabel (8) mit bestimmter Länge und Aussendurchmesser und mit einem Elektrodenkopf (9), der am distalen Ende des Elektrodenkabels angebracht ist, wobei die Anordnung eine im Vergleich zur Länge des Elektrodenkabels (8) relativ kurze und steife erste Hülse (1) umfasst, deren Innendurchmesser etwas grösser als der Aussendurchmesser des Elektrodenkabels (8) ist, wobei die eine Stirnseite der Hülse (1) eine Schneide (2) bildet und wobei die Hülse (1) mit von der Schneide (2) abgewandten ersten Steuermitteln (3) in Form eines Mandrins fest verbunden ist, mit dessen Hilfe die Hülse (1) auf das Elektrodenkabel (8) aufgeschoben und an der implantierten Elektrodenvorrichtung (7) entlang verschoben werden kann.

2. Anordnung nach Anspruch 1, **dadurch gekennzeichnet,** dass die Steuermittel (3) aus rostfreiem Stahl bestehen.

3. Anordnung nach Anspruch 1 oder 2, **gekennzeichnet** durch eine zweite Hülse (4), die auf die erste Hülse (1) aufschiebbar ist, wobei die eine Stirnseite der zweiten Hülse (4) mit federnden, in der Weise vorgebogenen Schneidemitteln (5) versehen ist, dass diese in einer ersten Lage gegen die Aussenwand der ersten Hülse (1) anliegen und in einer zweiten Lage gegeneinander gedrückt werden, wobei die zweite Hülse (4) mit von den Schneidemitteln (5) abgewandten zweiten Steuermitteln (6) verbunden ist, mit deren Hilfe die zweite Hülse (4) entlang der ersten Hülse (1) verschoben werden kann, so dass die Schneidemittel (5) von der einen in die andere Lage verschoben werden können.

4. Anordnung nach Anspruch 3, **dadurch gekennzeichnet**, dass die Schneidemittel (5) über den Umfang der zweiten Hülse (4) gleich verteilt sind.

5. Anordnung nach Anspruch 3 oder 4, **dadurch gekennzeichnet,** dass die zweiten Steuermittel (6) aus einem Rohr gebildet sind.

6. Anordnung nach einem der Ansprüche 3-5, **dadurch gekennzeichnet**, dass mindestens eine Hülse (1, 4) aus Metall besteht.

## Revendications

1. Dispositif pour explanter un dispositif (7) à électrode implanté, consistant en un dispositif (7) d'électrodes appliquées de manière intravasculaire ou intracardiaque, comportant un câble (8) d'électrode d'une certaine longueur et d'un certain diamètre extérieur et comportant une tête (9) d'électrode qui est montée à l'extrémité distale du câble d'électrode, le dispositif comprenant un premier manchon (1) qui est relativement court et rigide par rapport à la longueur du câble (8) d'électrode et dont le diamètre intérieur est un petit peu plus grand que le diamètre extérieur du câble (8) d'électrode, l'une des faces frontales du manchon (1) formant une lame (2) et le manchon (1) étant relié rigidement à des premiers moyens (3) de commande qui sont éloignés de la lame (2) et qui sont en forme d'un mandrin à l'aide duquel le manchon (1) peut être enfilé sur le câble (8) d'électrode et peut être déplacé le long du dispositif (7) à électrode implanté.

2. Dispositif suivant la revendication 1, caractérisé en ce que les moyens (3) de commande sont en acier inoxydable.

3. Dispositif suivant la revendication 1 ou 2, caractérisé par un deuxième manchon (4) qui peut être enfilé sur le premier manchon (1), l'une des faces frontales du deuxième manchon (4) étant munie de moyens (5) de découpe élastique, pré-incurvés de telle manière que ces moyens (5) s'appliquent à la paroi extérieure du premier manchon (1) en une première position et sont repoussés les uns sur les autres en une deuxième position, le deuxième manchon (4) étant relié à des deuxièmes moyens (6) de commande qui sont éloignés des moyens (5) de découpe et à l'aide desquels le deuxième manchon (4) peut être déplacé le long du premier manchon (1), si bien que les moyens (5) de découpe peuvent être déplacés d'une position à l'autre.

4. Dispositif suivant la revendication 3, caractérisé en ce que les moyens (5) de découpe sont répartis uniformément sur le pourtour du deuxième manchon (4).

5. Dispositif suivant la revendication 3 ou 4, caractérisé en ce que les deuxièmes moyens (6) de commande sont formés par un tuyau.

6. Dispositif suivant l'une des revendications 3 à 5, caractérisé en ce qu'au moins un manchon (1, 4) est en métal.
